# FASCICULE DE BREVET EUROPEEN

(11) **EP 0 804 606 B1**
(45) Date de publication et mention de la délivrance du brevet: **16.06.1999**
(21) Numéro de dépôt: 96901412.5
(22) Date de dépôt: 18.01.1996
(51) Int. Cl.: C12P 7/24, C12P 19/44, C07C 47/58, C07H 15/203

(54) **PROCEDE DE PREPARATION DE SUBSTANCES AROMATIQUES PAR VOIE BIOCHIMIQUE**
VERFAHREN ZUR BIOCHEMISCHEN GEWINNUNG VON AROMATISCHEN SUBSTANZEN
BIOCHEMICAL PROCESS FOR PREPARING AROMATIC SUBSTANCES

(30) Priorité: 19.01.1995 FR 9500581
(43) Date de publication de la demande: 05.11.1997
(73) Titulaire: ETABLISSEMENTS V. MANE FILS, 06620 Le-Bar-sur-Loup (FR)
(72) Inventeur: MANE, Jean, F-06130 Grassesse (FR); ZUCCA, Joseph, F-06600 Antibes (FR)
(74) Mandataire: Boulinguiez, Didier
(86) Numéro de dépôt international: FR9600082
(87) Numéro de publication internationale: WO9622381

(56) Documents cités:
- EP-A- 0 542 348
- WO-A-94/02621
- CHEMICAL ABSTRACTS, vol. 114, no. 3, 21 Janvier 1991 Columbus, Ohio, US; abstract no. 22487, YOSHIMOTO, TOMOTAKA ET AL: "Manufacture of benzaldehydes from styrenes with dioxygenase from Pseudomonas species" XP002002045 & JP,A,02 200 192 (HASEGAWA, T., CO., LTD., JAPAN)
- CHEMICAL ABSTRACTS, vol. 114, no. 11, 18 Mars 1991 Columbus, Ohio, US; abstract no. 99983, YOSHIMOTO, TOMOTAKA ET AL: "Dioxygenase for styrene cleavage manufactured by Pseudomonas" XP002002046 & JP,A,02 195 871 (HASEGAWA, T., CO., LTD., JAPAN)

## Description

L'invention a pour objet un procédé de préparation de certaines substances aromatiques par voie biochimique.

Par substances aromatiques, on entend des aldéhydes naturels présentant un intérêt aromatique, c'est-à-dire un parfum et/ou un goût propre à leur utilisation dans les industries alimentaire, pharmaceutique ou de la parfumerie.

Différents procédés de préparation des aldéhydes naturels ont déjà été décrits. Ainsi :
- le brevet US n° 4 617 419 décrit un procédé de préparation du benzaldéhyde et de l'acétaldéhyde au moyen d'une réaction "rétro-aldol",
- des phénylaldéhydes sont préparés par voie enzymatique dans la demande de brevet européen n° 542 348,
- les brevets US n° 5 017 388, US n° 5 128 253 et la demande de brevet européen n° 453 368 décrivent des procédés de préparation de la vanilline faisant intervenir des microorganismes,
- le brevet américain n° 5 057 424 décrit un procédé de préparation de compositions aromatiques de vanille au moyen de cellules végétales.

Ces procédés nécessitent cependant la mise en oeuvre de composés ou produits spécifiques et onéreux devant être mis en oeuvre dans des conditions relativement strictes.

La demande de brevet WO 94/02621 décrit un procédé de synthèse de composés aromatiques par voie enzymatique à partir de substrats identiques à ceux du présent brevet.

La présente invention a pour but de remédier aux inconvénients de la technique et permet la préparation de substances aromatiques au moyen de composés faciles à se procurer, peu coûteux et dans des conditions réactionnelles faciles à réaliser.

La Société demanderesse a en effet eu le mérite d'établir qu'il était possible de préparer des substances aromatiques de formule: 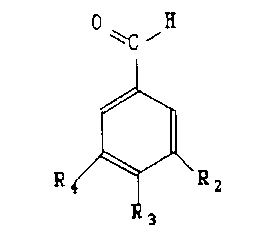 en soumettant à une oxydation, un substrat répondant à la formule 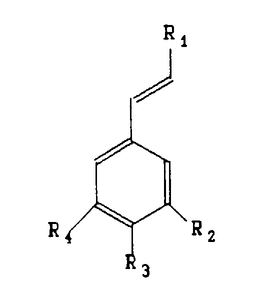 dans laquelle
R₁ peut être un radical -H, -CH₃, -CH₂OH, -CHO, -COOH, -OCH₃, ou -COO-CH(COOH)-CH₂-C₆H₃(OH)₂,
R₂ peut être un radical -H, -OH, ou -OCH₃,
R₃ peut être un radical -H, -OH, -OCH₃, ou O-glucoside,
R₄ peut être un radical -H, -OH, ou -OCH₃.
ladite oxydation étant conduite en présence d'au moins une protéine et de 0,0005 à 0,2% en poids par rapport au substrat d'un ion métallique qui se trouve sous forme libre ou complexée et qui est choisi dans le groupe comprenant le fer, le cobalt, le cuivre, le magnésium, le manganèse et le zinc.

En ce qui concerne le radical R₃, le glucoside du radical O-glucoside est choisi dans le groupe comprenant le glucopyranoside seul ou lié à un oside tel que le glucopyranoside, le rhamnopyranoside, l'arabinopyranoside.

Avantageusement, les substances aromatiques conformes à l'invention appartiennent au groupe comprenant la vanilline, l'aldéhyde benzoïque, l'aldéhyde anisique et l'aldéhyde protocatéchique.

La protéine mise en oeuvre dans le procédé conforme à l'invention peut être choisie parmi :
- les métalloprotéines, c'est-à-dire :
   . des protéines ou des enzymes ayant une structure hémique ; parmi elles, on peut citer la myoglobine, l'hémoglobine, les cytochromes (c, P450...) ainsi que de nombreuses oxydoréductases comme la peroxydase, la catalase, la cytochrome oxydase, ou des deshydrogénases comme l'alcool deshydrogénase, ou encore des oxygénases,
   . des protéines ou des enzymes associées à un ion métallique, parmi elles on peut citer la ferritine ainsi que la ferredoxine et certaines dioxygénases,
- des protéines dites "simples" dont l'action, dans le cadre de la présente invention, doit être complétée par un ion métallique non compris dans leur structure, cet ion métallique pouvant se trouver soit sous forme libre soit sous forme complexée (par exemple à des porphyrines ou à des protoporphyrines) ; parmi elles, on peut citer la gélatine, la caséine, la sérum albumine bovine.

Quelle que soit la protéine mise en oeuvre dans le procédé conforme à l'invention, l'ion métallique, sous forme libre ou complexée, est choisi dans le groupe comprenant le fer, le cobalt, le cuivre, le magnésium, le manganèse et le zinc.

Avantageusement, plusieurs protéines et plusieurs ions métalliques, sous forme libre ou complexée, peuvent simultanément être mis en oeuvre dans le procédé conforme à l'invention. En effet, il a été constaté que les rendements obtenus étaient d'autant plus élevés qu'ils résultaient de la mise en oeuvre de la combinaison d'au moins deux des composés suivants : une protéine "simple", une métalloprotéine, un ion métallique, une protoporphyrine, une porphyrine.

Le procédé conforme à l'invention présente notamment l'avantage de pouvoir être mis en oeuvre au moyen de composés peu coûteux et faciles à se procurer. De plus, les conditions réactionnelles ne nécessitent aucune précaution particulière de stérilité ou de régulation du milieu réactionnel.

Selon un mode de réalisation avantageux du procédé conforme à l'invention, l'ion métallique, sous quelque forme qu'il soit, est utilisé à raison de 0,0005 à 0,2 % en poids par rapport au substrat.

Avantageusement, les substrats soumis à l'action conjointe d'une protéine et d'un ion métallique sont choisis dans le groupe comprenant l'isoeugénol, l'eugénol, l'aldéhyde cinnamique, l'anéthol et l'acide rosmarinique.

Les protéines mises en oeuvre dans le procédé conforme à l'invention peuvent être utilisées sous forme purifiée ou non purifiée. Sous une forme non purifiée, ces protéines peuvent être mises en oeuvre par le biais de compositions les contenant telles qu'une suspension de cellules microbiennes, d'un broyat de cellules, de tissus ou d'organes d'origine végétale ou animale, ou encore d'un préconcentré protéique de ces compositions appelé fraction acétonique. A titre d'exemple on peut citer :
- des cellules de bactéries de levure ou de moisissure,
- des broyats végétaux tels que de fruits d'aubergines, de fruits de tomates, de feuilles d'épinards ou de fanes de radis,
- des extraits animaux, tels que du sang frais, du sang séché ou des cellules du foie.

Selon un mode de réalisation avantageux du procédé conforme à l'invention, la protéine, sous quelque forme qu'elle soit, est utilisée à raison de 0,01 à 20% en poids par rapport au substrat, de préférence à raison de 0,1 à 5 % en poids par rapport au substrat.

Le procédé conforme à l'invention peut être mis en oeuvre dans de l'eau ou dans un milieu aqueux tamponné à l'aide d'un tampon phosphate, citrate, borate ou phosphate-citrate-borate, le pH de la réaction devant être compris entre 2 et 10.

Avantageusement, dans le cas où le substrat est l'isoeugénol, l'anéthol ou l'aldéhyde cinnamique, le pH est compris de préférence entre 4 et 9 et plus préférentiellement entre 5 et 7 ; dans le cas où le substrat est l'eugénol, le pH est de préférence compris entre 2 et 7 et plus préférentiellement entre 2,5 et 4,5.

Le procédé conforme à l'invention peut également être mis en oeuvre dans un milieu non aqueux tel que dans un ou plusieurs solvants organiques choisis parmi : l'hexane, le cyclohexane, le méthylcyclohexane, le dichloroéthane, le dichlorométhane.

Par ailleurs, dans le cas où le substrat est l'eugénol, et ceci en raison de la très grande réactivité de ses groupements SH, l'ajout d'un composé sulphydryl peut être favorable. Ce composé peut être choisi dans le groupe comprenant la cystéine, le glutathion, le dithiothreitol, le dithioérythritol.

Selon d'autres caractéristiques avantageuses de l'invention :
- la température du milieu de réaction est comprise entre 15 et 50°C et de préférence entre 25 et 40°C,
- l'aération du milieu de réaction doit permettre la fourniture d'au moins une mole d'oxygène par mole de substrat à oxyder, de ce fait la réaction est réalisable à la pression atmosphérique, mais peut donner lieu à des rendements améliorés si le milieu réactionnel est enrichi en air ou en oxygène par exemple par simple bullage ; la réaction est également réalisable sous une pression comprise entre 10³ et 10⁷ pascals,
- le milieu réactionnel est soumis à une agitation vigoureuse pouvant être comprise entre 150 et 250 tr/min sur table d'agitation et entre 100 et 1000 tr/min en réacteur agité,
- la durée de la réaction est comprise entre 2 heures et 5 jours,
- les substances aromatiques préparées sont isolées du milieu réactionnel par extraction ou par distillation.

L'invention ne se limite pas strictement au procédé décrit, elle en embrasse au contraire toutes les variantes.

Les exemples qui suivent permettront de mieux comprendre l'invention ; ils ne sont pas limitatifs et concernent les modes de réalisation avantageux de l'invention.

### EXEMPLE 1 : Préparation de vanilline

On prépare un milieu réactionnel de base devant servir de témoin comprenant :
- 20 g de tampon borate, 0,1 M, pH 6,5,
- 800 mg d'isoeugénol et
- 50 mg de Tween® 80.

Un milieu réactionnel conforme à l'invention est également préparé et comprend, outre le milieu réactionnel de base, de l'hémoglobine purifiée.

Les milieux réactionnels sont mis à incuber à 28°C, pendant 3 jours, en flacons placés sur une table agitée à 175 tr/min.

Le tableau I rend compte des résultats obtenus.

**TABLEAU I**

| Composition du milieu réactionnel | Vanilline obtenue en g/l | Rendement |
|---|---|---|
| milieu de base (témoin) | 1,27 | 100 % |
| milieu de base + hémoglobine purifiée (20 mg) | 1,55 | 120 % |

Il apparaît très clairement que l'ajout d'hémoglobine purifiée améliore nettement le rendement d'obtention de la vanilline.

### EXEMPLE 2 : Préparation de vanilline

Le milieu réactionnel de base devant servir de témoin est le même que celui de l'exemple 1.

Plusieurs milieux réactionnels conformes à l'invention sont préparés et comprennent, outre le milieu réactionnel de base décrit dans l'exemple 1, au moins un des composés suivants à la quantité indiquée :
- de la gélatine,
- du fer sous forme FeCl₂,
- de la protoporphyrine IX.

Les conditions réactionnelles sont identiques à celles indiquées dans l'exemple 1.

Les résultats obtenus sont résumés dans le tableau II.

**TABLEAU II**

| Composition du milieu réactionnel | Vanilline obtenue en g/l | Rendement |
|---|---|---|
| milieu de base (témoin) | 0,93 | 100 % |
| milieu de base + gélatine (20 mg) | 0,6 | 64,50 % |
| milieu de base + fer (0,1 mg) | 0,85 | 91,40 % |
| milieu de base + gélatine (20 mg) + fer (0,1 mg) | 1,35 | 145% |
| milieu de base + gélatine (20 mg) + protoporphyrine (20 mg) + fer (0,025 mg) | 4,59 | 493 % |
| milieu de base + gélatine (20 mg) + protoporphyrine (20 mg) + fer (0,125 mg) | 5,29 | 568 % |

Alors que le rendement obtenu au moyen du milieu de base + gélatine ou du milieu de base + fer est inférieur à celui obtenu avec le milieu de base seul (simple constatation), l'association gélatine + fer permet d'accroître considérablement la quantité de vanilline obtenue, ceci d'autant plus que la protoporphyrine est présente dans le milieu et que la quantité de fer dans le milieu est élevée.

### EXEMPLE 3 : Préparation de vanilline

Le milieu réactionnel de base devant servir de témoin est le même que celui de l'exemple 1.

Plusieurs milieux réactionnels conformes à l'invention sont préparés et comprennent, outre le milieu réactionnel de base décrit dans l'exemple 1, au moins un des composés suivants à la quantité indiquée :
- de l'hémoglobine purifiée,
- du fer sous forme FeCl₂,
- de la protoporphyrine IX.

Les conditions réactionnelles sont identiques à celles indiquées dans l'exemple 1.

Le tableau III présente les résultats obtenus.

**TABLEAU III**

| Composition du milieu réactionnel | Vanilline obtenue en g/l | Rendement |
|---|---|---|
| milieu de base (témoin) | 1,15 | 100 % |
| milieu de base + hémoglobine purifiée (20 mg) | 1,72 | 149 % |
| milieu de base + hémoglobine purifiée (20 mg) + protoporphyrine (20 mg) + fer (0,025 mg) | 3,56 | 309 % |
| milieu de base + hémoglobine purifiée (20 mg) + protoporphyrine (20 mg) + fer (0,125 mg) | 5,07 | 440 % |

L'effet de l'hémoglobine sur le rendement est potentialisé par la présence de la protoporphyrine et du fer, et ceci d'autant plus que la quantité de ce dernier est importante.

### EXEMPLE 4 : Préparation de vanilline

On prépare, dans un réacteur agité, un milieu réactionnel de base devant servir de témoin comprenant :
- 2 l de tampon borate, 0,1 M, pH 6,5,
- 80 g d'isoeugénol et
- 500 mg de Tween® 80.

Deux milieux réactionnels conformes à l'invention sont également préparés et comprennent, outre le milieu réactionnel de base, respectivement 1 g d'hémoglobine purifiée et 1 g de sang séché.

Les milieux réactionnels sont mis à incuber à 30°C, pendant 24 heures, en milieu agité à 600 tr/min, sous une pression de 4.10⁴ pascals d'oxygène.

Le tableau IV rassemble les résultats obtenus.

**TABLEAU IV**

| Composition du milieu réactionnel | Vanilline obtenue en g/l | Rendement |
|---|---|---|
| milieu de base (témoin) | 1,75 | 100 % |
| milieu de base + hémoglobine purifiée (1 g) | 3,45 | 196 % |
| milieu de base + sang séché (1 g) | 4,25 | 243 % |

Le sang séché, dont la composition comprend outre l'hémoglobine, des protéines plasmatiques (comme l'albumine et les globulines) et du fer, permet l'obtention de meilleurs résultats encore qu'avec l'hémoglobine purifiée.

### EXEMPLE 5 : Préparation de vanilline

On prépare, dans un réacteur agité, un milieu réactionnel de base devant servir de témoin comprenant :
- 2 l de tampon borate, 0,1 M, pH 6,5,
- 100 g d'isoeugénol et
- 500 mg de Tween® 80.

Un milieu réactionnel conforme à l'invention est également préparé et comprend, outre le milieu réactionnel de base, 2 g d'hémoglobine purifiée.

Les milieux réactionnels sont mis à incuber à 30°C, pendant 24 heures, en milieu agité à 600 tr/min, sous une pression d'oxygène de 5.10⁴ pascals.

Le tableau V rend compte des résultats obtenus.

**TABLEAU V**

| Composition du milieu réactionnel | Vanilline obtenue en g/l | Rendement |
|---|---|---|
| milieu de base (témoin) | 1,75 | 100 % |
| milieu de base + hémoglobine purifiée (2 g) | 8,4 | 480 % |

Ces résultats confirment les résultats obtenus dans les exemples précédents.

### EXEMPLE 6 : Préparation de vanilline

On prépare deux milieux réactionnels de base identiques comprenant :
- 25 g de tampon borate, 0,1 M, pH 6,5,
- 400 mg d'isoeugénol et
- 50 mg de Tween® 80.

L'un d'entre eux, considéré comme le milieu témoin, est additionné de 25 g de fruit d'aubergine broyé finement ayant subi au préalable un traitement thermique à 110°C pendant 15 minutes.

L'autre milieu est complémenté avec 25 g de fruit d'aubergine broyé finement et non traité thermiquement (fruit frais).

Il est connu que le fruit entier d'aubergine comprend des protéines à raison de 1 g par 100 g d'aubergine. Ainsi, dans 25 g d'aubergine, on a environ 250 mg de protéines (parmi lesquelles on compte certaines activités enzymatiques oxydatives) et environ 0,075 mg de fer et 3,75 mg de magnésium.

Les deux milieux sont mis à incuber à 28°C, pendant 3 jours, en flacons placés sur table agitée à 175 tr/min.

Le tableau VI présente les résultats obtenus.

**TABLEAU VI**

| Composition du milieu réactionnel | Vanilline obtenue en mg/l | Rendement |
|---|---|---|
| milieu de base témoin | 700 | 100 % |
| milieu de base + fruit frais (25 g) | 1750 | 250 % |

Ceci démontre que les broyats végétaux notamment permettent l'obtention d'excellents résultats dans le cadre de l'invention.

### EXEMPLE 7 : Préparation de vanilline

On prépare un milieu réactionnel de base devant servir de témoin comprenant :
- 20 g de tampon phosphate/citrate/borate, 0,1 M, pH 6,5,
- 800 mg d'eugénol et
- 50 mg de Tween® 80.

Deux milieux réactionnels conformes à l'invention sont également préparés et comprennent, outre le milieu réactionnel de base, respectivement de la cystéine et de la cystéine + de l'hémoglobine purifiée.

Les milieux réactionnels sont mis à incuber à 30°C, pendant 2 heures, en milieu agité à 150 tr/min, sous une pression d'oxygène de 8.10⁵ pascals.

Le tableau VII rend compte des résultats obtenus.

**TABLEAU VII**

| Composition du milieu réactionnel | Vanilline obtenue, en mg/l | Rendement |
|---|---|---|
| milieu de base (témoin) | 18 | 100 % |
| milieu de base + cystéine (0,6 g) | 177 | 983 % |
| milieu de base + cystéine (0,6 g) + hémoglobine purifiée (20 mg) | 703 | 3 950 % |

La grande réactivité de l'eugénol vis à vis de la cystéine se trouve ici démontrée et cette combinaison est considérablement potentialisée par l'ajout d'hémoglobine purifiée.

### EXEMPLE 8 : Préparation de vanilline

On prépare un milieu réactionnel de base, devant servir de témoin, comprenant :
- 20 g de tampon phosphate/citrate/borate, 0,1 M, pH 6,5,
- 800 mg d'eugénol,
- 50 mg de Tween® 80.

Un milieu réactionnel conforme à l'invention est également préparé et comprend, outre le milieu réactionnel de base :
- de la cystéine,
- de la gélatine,
- de la protoporphyrine IX et
- du fer sous forme FeSO₄.

Les milieux réactionnels sont mis à incuber à 30°C, pendant 2 heures, en milieu agité à 150 tr/min, sous une pression d'oxygène de 10⁵ pascals.

Les résultats obtenus sont résumés dans le tableau VIII.

**TABLEAU VIII**

| Composition du milieu réactionnel | Vanilline obtenue en mg/l | Rendement |
|---|---|---|
| milieu de base (témoin) | 18 | 100 % |
| milieu de base + cystéine (0,6 g) + gélatine (20 mg) + protoporphyrine IX (20 mg) + fer (0,125 mg) | 556 | 3 088 % |

Ici, d'excellents résultats sont encore obtenus et la réactivité de l'eugénol vis à vis de la cystéine est renforcée par la présence de la gélatine, de la protoporphyrine IX et du fer.

### EXEMPLE 9 : Préparation de benzaldéhyde

On prépare, dans un réacteur agité, un milieu réactionnel de base devant servir de témoin comprenant :
- 2 l de tampon borate, 0,1 M, pH 6,5,
- 82 g d'aldéhyde cinnamique et
- 1 g de Tween® 80.

Un milieu réactionnel conforme à l'invention est également préparé et comprend, outre le milieu réactionnel de base :
- de l'hémoglobine purifiée,
- du fer sous forme FeCl₂.

Les milieux réactionnels sont mis à incuber à 30°C, pendant 48 heures, en milieu agité à 600 tr/min, sous une pression d'oxygène de 10⁴ pascals.

Le tableau IX présente les résultats obtenus.

**TABLEAU IX**

| Composition du milieu réactionnel | Benzaldéhyde en mg/l | Rendement |
|---|---|---|
| milieu de base (témoin) | 500 | 100 % |
| milieu de base + hémoglobine purifiée (2 g) + fer (10 mg) | 4 220 | 844 % |

L'hémoglobine et le fer potentialisent de façon spectaculaire la réaction d'oxydation de l'aldéhyde cinnamique en benzaldéhyde.

### EXEMPLE 10 : Préparation d'anisaldéhyde

On prépare, dans un réacteur agité, un milieu réactionnel de base devant servir de témoin comprenant :
- 2 l de tampon borate, 0,1 M, pH 6,5,
- 80 g d'anéthol et
- 1 g de Tween® 80.

Un milieu réactionnel conforme à l'invention est également préparé et comprend, outre le milieu réactionnel de base :
- de l'hémoglobine purifiée,
- du fer sous forme FeCl₂.

Les milieux réactionnels sont mis à incuber à 30°C, pendant 48 heures, en milieu agité à 600 tr/min, sous une pression d'oxygène de 10⁴ pascals.

Le tableau X présente les résultats obtenus.

**TABLEAU X**

| Composition du milieu réactionnel | Anisaldéhyde en g/l | Rendement |
|---|---|---|
| milieu de base (témoin) | 1,7 | 100 % |
| milieu de base + hémoglobine purifiée (2 g) + fer (10 mg) | 14 | 823 % |

L'hémoglobine et le fer potentialisent de façon spectaculaire la réaction d'oxydation de l'anéthol en anisaldéhyde.

Dans les EXEMPLES 11 à 13, les conditions réactionnelles sont les mêmes et sont exposées ci-après.

On prépare, dans un réacteur agité, un milieu réactionnel de base devant servir de témoin comprenant :
- 20 g de tampon borate,0,1 M, pH 6,5,
- 800 mg de substrat,
- 50 mg de Tween® 80.

Un milieu réactionnel conforme à l'invention est également préparé et comprend, outre le milieu réactionnel de base :
- de l'hémoglobine purifiée ou
- de l'hémoglobine purifiée et du fer sous forme FeCl₂.

Les milieux réactionnels sont mis à incuber à 28°C, pendant 2 heures, en milieu agité à 150 tr/min, sous une pression d'oxygène de 5.10⁴ pascals. Les tableaux XI à XIII présentent les résultats obtenus.

### EXEMPLE 11 : Préparation de vanilline

Le substrat est l'isoeugénol.

**TABLEAU XI**

| Composition du milieu réactionnel | Vanilline obtenue en g/l | Rendement |
|---|---|---|
| milieu de base (témoin) | 0,42 | 100 % |
| milieu de base + hémoglobine purifiée (20mg) | 0,5 | 119 % |
| milieu de base + hémoglobine purifiée (20 mg) + fer (0,1 mg) | 0,53 | 126 % |

L'effet de l'hémoglobine purifiée sur le rendement est clairement potentialisé par la présence du fer.

### EXEMPLE 12 : Préparation d'aldéhyde anisique

Le substrat est l'anéthol.

**TABLEAU XII**

| Composition du milieu réactionnel | Anisaldéhyde obtenu en g/l | Rendement |
|---|---|---|
| milieu de base (témoin) | 0,196 | 100 % |
| milieu de base + hémoglobine purifiée (20mg) | 0,188 | 96 % |
| milieu de base + hémoglobine purifiée (20 mg) + fer (0,1 mg) | 0,244 | 124 % |

Nous ne pouvons que constater une diminution (non expliquée) du rendement dans le cas où l'hémoglobine purifiée est ajoutée au milieu de base mais la combinaison hémoglobine purifiée-fer permet l'obtention d'un rendement en accord avec celui obtenu dans l'exemple précédent.

### EXEMPLE 13 : Préparation de benzaldéhyde

Le substrat est l'aldéhyde cinnamique.

**TABLEAU XIII**

| Composition du milieu réactionnel | Benzaldéhyde obtenu en g/l | Rendement |
|---|---|---|
| milieu de base (témoin) | 0,089 | 100 % |
| milieu de base + hémoglobine purifiée (20mg) | 0,809 | 909 % |
| milieu de base + hémoglobine purifiée (20 mg) + fer (0,1 mg) | 0,833 | 936 % |

L'hémoglobine purifiée potentialise de façon spectaculaire la réaction d'oxydation de l'aldéhyde cinnamique en benzaldéhyde, cette réaction étant d'autant plus renforcée par la présence du fer.

### EXEMPLE 14 : Préparation de vanilline

On prépare un milieu réactionnel de base, devant servir de témoin, comprenant :
- 20 g de tampon borate, 0,1 M, pH 6,5,
- 800 mg d'isoeugénol,
- 50 mg de Tween 80®.

Un milieu réactionnel conforme à l'invention est également préparé et comprend, outre le milieu réactionnel de base :
- de l'hémoglobine purifiée ou
- de l'hémoglobine purifiée et du fer sous forme FeCl₂.

Les milieux réactionnels sont mis à incuber à 28° C, pendant 2 heures, en milieu agité à 150 tr/min, sous une pression d'oxygène de 8.10⁵ pascals.

Les résultats obtenus sont résumés dans le tableau XIV.

**TABLEAU XIV**

| Composition du milieu réaction ne | Vanilline obtenue en g/l | Rendement |
|---|---|---|
| milieu de base (témoin) | 3 | 100 % |
| milieu de base + hémoglobine purifiée (20mg) | 3,57 | 119 % |
| milieu de base + hémoglobine purifiée (20 mg) + fer (0,1 mg) | 5,69 | 190 % |

L'ajout de fer au milieu de base en plus de l'hémoblogine purifiée renforce de façon substantielle le rendement d'obtention de la vanilline par rapport au rendement, déjà intéressant, obtenu dans le cas où le milieu réactionnel ne se compose que du milieu de base et de l'hémoglobine purifiée.

## Revendications

1. Procédé de préparation de substances aromatiques de formule 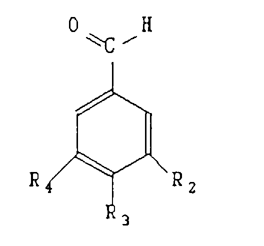 caractérisé par le fait que l'on soumet à une oxydation un substrat répondant à la formule : 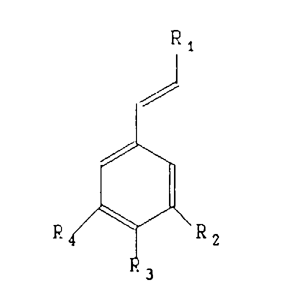 dans laquelle
R₁ peut être un radical -H, -CH₃, -CH₂OH, -CHO, -COOH, -OCH₃, ou -COO-CH(COOH)-CH₂-C₆H₃(OH)₂,
R₂ peut être un radical -H, -OH, ou -OCH₃,
R₃ peut être un radical -H, -OH, -OCH₃, ou O-glucoside,
R₄ peut être un radical -H, -OH, ou -OCH₃,
ladite oxydation étant conduite en présence d'au moins une protéine et de 0,0005 à 0,2% en poids par rapport au substrat d'un ion métallique qui se trouve sous forme libre ou complexée et qui est choisi dans le groupe comprenant le fer, le cobalt, le cuivre, le magnésium, le manganèse et le zinc.

2. Procédé selon la revendication 1, caractérisé par le fait que le glucoside dans le radical R₃ est choisi dans le groupe comprenant le glucopyranoside seul ou lié à un oside tel que le glucopyranoside, le rhamnopyranoside, l'arabinopyranoside.

3. Procédé selon la revendication 1 ou 2, caractérisé par le fait que les substances aromatiques appartiennent au groupe comprenant la vanilline, l'aldéhyde benzoïque, l'aldéhyde anisique, l'aldéhyde protocatéchique.

4. Procédé selon l'une des revendications 1 à 3, caractérisé par le fait que la protéine mise en oeuvre est une métalloprotéine

5. Procédé selon la revendications 4, caractérisé par le fait que la métalloprotéine est choisie dans le groupe comprenant l'hémoglobine, la myoglobine, les oxydoréductases, les deshydrogénases, les oxygénases.

6. Procédé selon l'une des revendications 1 à 3, caractérisé par le fait que la protéine est choisie dans le groupe comprenant la gélatine, la caséine, la sérum albumine bovine.

7. Procédé selon l'une des revendications 1 à 6, caractérisé par le fait que la protéine est utilisée sous forme purifiée.

8. Procédé selon l'une des revendications 1 à 6, caractérisé par le fait que la protéine est utilisée sous forme non purifiée au moyen d'une suspension de cellules microbiennes, d'un broyat de cellules, de tissus ou d'organes d'origine végétale ou animale ou d'un préconcentré protéique de ces compositions.

9. Procédé selon l'une des revendications 1 à 8, caractérisé par le fait que la protéine est utilisée à raison de 0,01 à 20% en poids par rapport au substrat de préférence à raison de 0,1 à 5 % en poids par rapport au substrat.

10. Procédé selon l'une des revendications 1 à 9, caractérisé par le fait qu'il est mis en oeuvre dans de l'eau ou dans un milieu aqueux tamponné de pH compris entre 2 et 10.

11. Procédé selon l'une des revendications 1 à 10, caractérisé par le fait qu'il est mis en oeuvre à une température comprise entre 15 et 50°C et de préférence entre 25 et 40°C.

12. Procédé selon l'une des revendications 1 à 11, caractérisé par le fait qu'il est mis en oeuvre à la pression atmosphérique ou sous une pression d'air ou d'oxygène comprise entre 10³ et 10⁷ pascals.

13. Procédé selon l'une des revendications 1 à 12, caractérisé par le fait qu'il est mis en oeuvre sous agitation.

14. Procédé selon l'une des revendications 1 à 13, caractérisé par le fait qu'il est mis en oeuvre pendant une durée comprise entre 2 heures et 5 jours.

15. Procédé selon l'une des revendications 1 à 14, caractérisé par le fait que les substances aromatiques préparées sont isolées du milieu réactionnel par extraction ou par distillation.

## Patentansprüche

1. Verfahren zur Herstellung aromatischer Substanzen nach der Formel 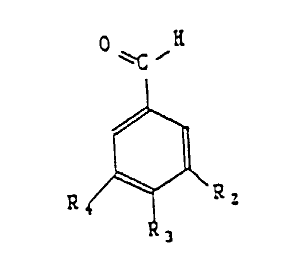 dadurch gekennzeichnet, dass man ein der folgenden Formel entsprechendes Substrat einer Oxidation unterzieht: 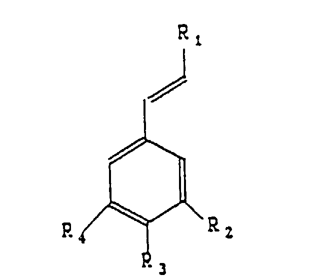 worin **R**_{**1**} ein Radikal -H, -CH₃, -CH₂OH, -CHO, -COOH, -OCH₃ oder -COO-CH(COOH)-CH₂-C₆H₃(OH)₂ sein kann, **R**_{**2**} ein Radikal -H, -OH oder -OCH₃ sein kann, **R**_{**3**} ein Radikal -H, -OH, -OCH₃ oder O-Glucosid sein kann, **R**_{**4**} ein Radikal -H, -OH oder -OCH₃ sein kann, wobei die genannte Oxidation in Gegenwart mindestens eines Proteins und, im Verhältnis zum Substrat, 0,0005 bis 0,2 Gewichts-% eines Metallions durchgeführt wird, wobei das Metallion in freier oder komplexer Form vorliegt und aus der Gruppe bestehend aus Eisen, Kobalt, Kupfer, Magnesium, Mangan oder Zink gewählt wird.

2. Verfahren nach Anspruch 1, dadurch gekennzeichnet, dass das Glucosid in dem Radikal R₃ aus der Gruppe ausgewählt wird, die aus Glucopyranosid allein oder Verbindungen mit einem Osid besteht, wie zum Beispiel Glucopyranosid, Rhamnopyranosid, Arabinopyranosid.

3. Verfahren nach Anspruch 1 oder 2, dadurch gekennzeichnet, dass die aromatischen Substanzen zu der Gruppe gehören, die Vanillin, Benzaldehyd, Anisaldehyd, Protokatechusäure enthält.

4. Verfahren nach einem der Ansprüche 1 bis 3, dadurch gekennzeichnet, dass das zum Einsatz kommende Protein ein Metallprotein ist.

5. Verfahren nach Anspruch 4, dadurch gekennzeichnet, dass das Metallprotein aus der Gruppe ausgewählt wird, die Hämoglobin, Myoglobin, Oxidoreduktasen, Deshydrasen, Oxygenasen enthält.

6. Verfahren nach einem der Ansprüche 1 bis 3, dadurch gekennzeichnet, dass das Protein aus der Gruppe ausgewählt wird, die Gelatine, Kasein, Rindereiweiß-Serum enthält.

7. Verfahren nach einem der Ansprüche 1 bis 6, dadurch gekennzeichnet, dass das Protein in gereinigter Form verwendet wird.

8. Verfahren nach einem der Ansprüche 1 bis 6, dadurch gekennzeichnet, dass das Protein in ungereinigter Form verwendet wird, unter Verwendung einer Lösung von mikrobischen Zellen, einem Zellbrei, Geweben oder Teilen pflanzlichen oder tierischen Ursprungs oder einem Proteinkonzentrat aus diesen Stoffen.

9. Verfahren nach einem der Ansprüche 1 bis 8, dadurch gekennzeichnet, dass das Protein in einem Verhältnis von 0,01 bis 20 Gewichts-% im Verhältnis zum Substrat, vorzugsweise in einem Verhältnis von 0,1 bis 5 Gewichts-% im Verhältnis zum Substrat verwendet wird.

10. Verfahren nach einem der Ansprüche 1 bis 9, dadurch gekennzeichnet, dass es in Wasser oder in einem wässrigen Milieu mit einem gepufferten pH-Wert zwischen 2 und 10 in Gang gesetzt wird.

11. Verfahren nach einem der Ansprüche 1 bis 10, dadurch gekennzeichnet, dass es bei einer Temperatur zwischen 15° und 50°C, vorzugsweise zwischen 25° und 40°C in Gang gesetzt wird.

12. Verfahren nach einem der Ansprüche 1 bis 11, dadurch gekennzeichnet, dass es unter atmosphärischem Druck oder einem Luft- oder Sauerstoffdruck zwischen 10³ und 10⁷ Pascal in Gang gesetzt wird.

13. Verfahren nach einem der Ansprüche 1 bis 12, dadurch gekennzeichnet, dass es unter Bewegung in Gang gesetzt wird.

14. Verfahren nach einem der Ansprüche 1 bis 13, dadurch gekennzeichnet, dass es eine Zeitspanne zwischen 2 Stunden und 5 Tagen umfaßt.

15. Verfahren nach einem der Ansprüche 1 bis 14, dadurch gekennzeichnet, dass die bereiteten aromatischen Substanzen aus dem Reaktionsmedium durch Extraktion oder Destillation isoliert werden.

## Claims

1. A process for the preparation of aromatic substances having the formula 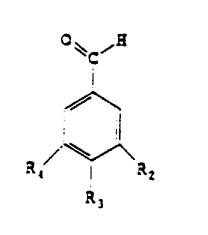 characterised in that a substrate corresponding to the formula 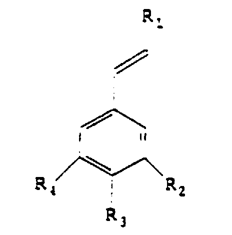 is subjected to oxidation, a formula wherein
R₁ may be a radical -H, -CH₃, -CH₂OH, -CHO, -COOH, -OCH₃ or -COO-CH(COOH)-CH₂-C₆H₃(OH)₂,
R₂ may be a radical -H, -OH, or -OCH₃,
R₃ may be a radical -H, -OH, -OCH₃, or 0-glucoside,
R₄ may be a radical -H, -OH, or -OCH₃.
said oxidation being carried out in the presence of at least one protein and of 0.0005 to 0.2% by weight with respect to the substrate of a metal ion which is in the free or complexed form and which is chosen from the group comprising iron, cobalt, copper, magnesium, manganese and zinc.

2. A process according to claim 1, characterised in that the glucoside in the radical R₃ is chosen from the group comprising glucopyranoside alone or attached to an oside such as glucopyranoside, rhamnopyranoside, arabinopyranoside.

3. A process according to claim 1 or 2, characterised in that the aromatic substances belong to the group comprising vanillin, benzaldehyde, anisaldehyde, protocatechualdehyde.

4. A process according to one of claims 1 to 3, characterised in that the protein used is a metalloprotein.

5. A process according to claim 4, characterised in that the metalloprotein is chosen from the group comprising haemoglobin, myoglobin, oxydoreductases, dehydrogenases, oxygenases.

6. A process according to one of claims 1 to 3, characterised in that the protein is chosen from the group comprising gelatin, casein, bovine serum albumin.

7. A process according to one of claims 1 to 6, characterised in that the protein is used in the purified form.

8. A process according to one of claims 1 to 6, characterised in that the protein is used in the unpurified form by means of a suspension of microbial cells, a ground preparation of cells, tissues or organs of vegetable or animal origin or of a protein preconcentrate of said compositions.

9. A process according to one of claims 1 to 8, characterised in that the protein is used in a quantity of 0.01 to 20% by weight with respect to the substrate, preferably in a quantity of 0.1 to 5% by weight with respect to the substrate.

10. A process according to one of claims 1 to 9, characterised in that it is used in water or in a buffered aqueous medium having a pH between 2 and 10.

11. A process according to one of claims 1 to 10, characterised in that it is used at a temperature between 15 and 50°C and preferably between 25 and 40°C.

12. A process according to one of claims 1 to 11, characterised in that it is used at atmospheric pressure or under a pressure of air or oxygen between 10³ and 10⁷ pascals.

13. A process according to one of claims 1 to 12, characterised in that it is used under agitation.

14. A process according to one of claims 1 to 13, characterised in that it is used for a period between 2 hours and 5 days.

15. A process according to one of claims 1 to 14, characterised in that the aromatic substances prepared are isolated from the reaction medium by extraction or by distillation.
